# FASCICULE DE BREVET EUROPEEN

(11) **EP 0 690 836 B1**
(45) Date de publication et mention de la délivrance du brevet: **19.08.1998**
(21) Numéro de dépôt: 94909156.5
(22) Date de dépôt: 07.03.1994
(51) Int. Cl.: C07C 51/353, C07C 55/14

(54) **PROCEDE D'ISOMERISATION D'ACIDES CARBOXYLIQUES**
VERFAHREN ZUR ISOMERISIERUNG VON CARBONSÄUREN
CARBOXYLIC ACID ISOMERISATION METHOD

(30) Priorité: 25.03.1993 FR 9303703
(43) Date de publication de la demande: 10.01.1996
(73) Titulaire: RHODIA FIBER & RESIN INTERMEDIATES, 92408 Courbevoie Cédex (FR)
(72) Inventeur: DENIS, Philippe, F-69150 Decines (FR); PATOIS, Carl, F-69003 Lyon (FR); PERRON, Robert, F-69390 Charly (FR)
(74) Mandataire: Vignally, Noel
(86) Numéro de dépôt international: FR9400247
(87) Numéro de publication internationale: WO9421586

(56) Documents cités:
- US-A- 3 090 807
- US-A- 3 578 688
- US-A- 3 592 849

## Description

La présente invention concerne un procédé d'isomérisation d'acides carboxyliques, plus précisément d'acides carboxyliques saturés ramifiés, en acides linéaires correspondants.

L'hydroxycarbonylation des acides penténoïques pour préparer l'acide adipique conduit toujours à la formation de quantités plus ou moins importantes de diacides ramifiés, comme sous-produits de la réaction.

La valorisation de ces diacides ramifiés représente donc, notamment, un problème important à résoudre, dans le cadre d'un procédé industriel de préparation d'acide adipique par hydroxycarbonylation des acides penténoïques.

Dans le brevet EP-A-0 374 687, il est décrit un procédé d'isomérisation d'acides carboxyliques saturés, par chauffage sous une pression de monoxyde de carbone, en présence d'un catalyseur au rhodium et d'un promoteur iodé ou bromé.

La présente invention se caractérise par l'utilisation d'iridium, un catalyseur différent de celui qui a été décrit antérieurement pour cette réaction.

Plus spécifiquement, elle consiste en un procédé d'isomérisation par chauffage, d'au moins un acide carboxylique saturé ramifié, caractérisé en ce que l'on opère en présence d'une quantité efficace d'un catalyseur à l'iridium et d'un promoteur iodé ou bromé, le rapport molaire promoteur/lr étant compris entre 0,1/1 et 20/1.

Parmi les acides carboxyliques saturés ramifiés, les diacides ramifiés sont d'un point de vue économique les plus intéressants à valoriser, en raison des fortes quantités formées lors de l'hydroxycarbonylation des acides penténoïques pour la préparation de l'acide adipique.

Ce sont plus précisément l'acide 2-méthyl-glutarique, l'acide 2-éthyl-succinique et leurs mélanges entre eux et/ou avec d'autres acides carboxyliques ou lactones formés en même temps qu'eux, comme par exemple l'acide adipique, les acides penténoïques, l'acide valérique, la gamma-valérolactone.

Dans les mélanges précédents, les composés autres que l'acide 2-méthyl-glutarique et l'acide 2-éthyl-succinique peuvent représenter jusqu'à 50 % en poids du poids total du mélange.

Pour le catalyseur à l'iridium nécessaire dans le présent procédé, diverses sources d'iridium sont susceptibles d'être mises en oeuvre.

A titre d'exemples de telles sources d'iridium, on peut citer :
- Ir métallique; IrO₂ ; Ir₂O₃ ;
- IrCl₃ ; IrCl₃, 3H₂O ;
- IrBr₃ ; IrBr₃, 3H₂O ;
- Irl₃ ;
- Ir₂(CO)₄Cl₂ ; Ir₂(CO)₄l₂ ;
- Ir₂(CO)₈ ; Ir₄(CO)₁₂ ;
- Ir(CO)[P(C₆H₅)₃]₂l ;
- Ir(CO)[P(C₆H₅)₃]₂Cl ;
- Ir[P(C₆H₅)₃]₃l ;
- HIr[P(C₆H₅)₃]₃(CO) ;
- Ir(acac)(CO)₂ ;
- [IrCl(cod)]₂ ;
(acac = acétylacétonate ; cod = cyclooctadiène-1,5).

Les catalyseurs à l'iridium qui conviennent plus particulièrement sont: [lrcl(cod)]₂ , lr₄(CO)₁₂ et lr(acac)(CO)₂.

La quantité de catalyseur à mettre en oeuvre peut varier dans de larges limites.

En général, une quantité, exprimée en moles d'iridium métal par litre de mélange réactionnel, comprise entre 10⁻⁴ et 10⁻¹ conduit à des résultats satisfaisants. Des quantités inférieures peuvent être utilisées, mais on observe toutefois que la vitesse de réaction est faible. Des quantités supérieures n'ont des inconvénients qu'au plan économique.

De préférence, la concentration en iridium dans le mélange réactionnel est comprise entre 5.10⁻⁴ et 5.10⁻² mole/litre.

Par promoteur iodé ou bromé, on entend dans le cadre du procédé l'iodure d'hydrogène, le bromure d'hydrogène et les composés organo-iodés et organo-bromés capables de générer respectivement l'iodure d'hydrogène et le bromure d'hydrogène dans les conditions réactionnelles; ces composés organo-iodés et organo-bromés sont plus particulièrement les iodures et les bromures d'alkyle ayant de 1 à 10 atomes de carbone, parmi lesquels l'iodure de méthyle et le bromure de méthyle sont préférés.

Le rapport molaire promoteur/lr est de préférence compris entre 1/1 et 10/1.

La réaction est conduite en phase liquide. La température à laquelle on opère est généralement de 120°C à 300°C et de préférence de 150°C à 250°C.

Bien que l'on puisse réaliser l'isomérisation selon le procédé de l'invention en l'absence de monoxyde de carbone, il est préférable d'opérer en sa présence.

La pression totale à la température de la réaction peut alors varier dans de larges limites. La pression partielle du monoxyde de carbone, mesurée à 25°C, est généralement de 0,5 bar à 100 bars et de préférence de 1 bar à 80 bars.

Le monoxyde de carbone utilisé peut être le monoxyde de carbone sensiblement pur ou le monoxyde de carbone de qualité technique, tels qu'ils se trouvent dans le commerce.

La réaction d'isomérisation des acides carboxyliques saturés ramifiés est conduite soit dans l'acide lui-même comme milieu liquide de la réaction, soit dans un tiers solvant.

Comme tiers solvant, on peut utiliser notamment les acides carboxyliques aliphatiques, saturés ou insaturés, ou aromatiques, comportant au plus 20 atomes de carbone, dans la mesure où ils sont liquides dans les conditions réactionnelles. A titre d'exemples de tels acides carboxyliques, on peut citer l'acide acétique, l'acide propionique, l'acide butanoïque, l'acide valérique, l'acide adipique, les acides penténoïques, l'acide benzoïque et l'acide phénylacétique.

D'autres familles de solvants peuvent également être utilisés, notamment les hydrocarbures saturés aliphatiques ou cycloaliphatiques et leurs dérivés chlorés, dans la mesure où ils sont liquides dans les condition réactionnelles. A titre d'exemples de tels solvants, on peut citer le dichlorométhane, l'hexane et le cyclohexane. On peut encore citer le benzène, le toluène et le chlorobenzène.

Des mélanges de solvants peuvent être utilisés.

Lorsqu'il est présent dans le mélange réactionnel, le solvant représente par exemple de 10 % à 99 % en volume par rapport au volume total dudit mélange réactionnel et de préférence de 30 % à 90 % en volume.

L'eau est le plus souvent présente dans le mélange réactionnel. Généralement, elle représente de 0 à 20 % en poids du mélange réactionnel et de préférence de 0 à 10 % .

Lorsque la réaction d'isomérisation est conduite dans un solvant, une variante intéressante consiste à opérer dans un mélange eau/solvant miscible à l'eau, tel que par exemple eau/acide acétique. Dans une telle hypothèse, les valeurs des proportions du mélange eau/solvant miscible à l'eau dans le mélange réactionnel sont celles qui ont été indiquées précédemment pour le seul solvant.

Le procédé d'isomérisation selon l'invention permet de valoriser notamment les diacides carboxyliques saturés ramifiés obtenus en quantités plus ou moins importantes lors de l'hydroxycarbonylation des acides penténoïques. Cela permet une augmentation du rendement global d'un procédé de préparation d'acide adipique à partir du butadiène via les acides penténoïques, l'acide adipique étant l'une des matières premières de la préparation des polyamides 6-6.

Le mélange réactionnel final est traité selon les méthodes classiques utilisées en chimie, afin de séparer les différents composés formés et les acides saturés ramifiés n'ayant pas réagi, ces demiers pouvant être soumis à une nouvelle isomérisation.

Le procédé selon l'invention peut être mis en oeuvre en continu ou en discontinu. Lorsque l'on opère en continu, les rapports relatifs des réactifs, catalyseur, promoteur et solvant pourront être fixés à une valeur optimale par l'homme du métier, alors que généralement dans un procédé mis en oeuvre en discontinu, ces différents rapports évoluent en fonction de la transformation progressive des réactifs.

Les exemples qui suivent illustrent l'invention.

### EXEMPLE 1.

Dans un autoclave de 125 cm³, préalablement purgé à l'argon, on introduit successivement :
- [lrCl(cod)]₂: 0,84 mmol
- iodure d'hydrogène (en solution aqueuse à 57 % en poids) : 1,2 mmol
- acide 2-méthyl-glutarique : 39 mmol
- acide acétique : 40 cm³

L'autoclave est fermé, placé dans un four agité et raccordé à l'alimentation de CO sous pression. On met 5 bar de CO à 25°C, puis on chauffe à 230°C. On ajuste la pression en température à 25 bar à l'aide de CO (ce qui correspond à 17 bar de pression partielle de CO mesurée à 25°C) et cette pression est maintenue pendant 5 heures à 230°C.

L'autoclave est ensuite refroidi, puis dégazé et le mélange réactionnel est analysé par chromatographie en phase gazeuse (CPG) et en chromatographie liquide à haute performance (CLHP).

On obtient les résultats suivants :
- taux de transformation (TT) de l'acide 2-méthyl-glutarique: 18 %
- rendement (RT) molaire en acide adipique par rapport à l'acide méthyl-glutarique transformé: 37 %
- RT en acide 2-éthyl-succinique : 6 %
- RT en gamma-valérolactone: 6 %
- RT en acide 3-penténoïque: 10 %
- RT en acide 2-méthyl-butanoïque : 26 %
- RT en acide valérique : 16 %

### EXEMPLE 2.

On répète l'exemple 1, dans les mêmes conditions et avec les mêmes quantités des mêmes réactifs, mais en utilisant 2,5 mmol d'iodure d'hydrogène et 0,42 mmol de [lrCl(cod)]₂.

On obtient les résultats suivants :

| | |
|---|---|
| TT de l'acide 2-méthyl-glutarique | 36 % |
| RT en acide adipique | 28 % |

### EXEMPLE 3.

On répète l'exemple 1, dans les mêmes conditions et avec les mêmes quantités des mêmes réactifs, mais en utilisant 2,5 mmol d'iodure d'hydrogène et en opérant à 200°C.

On obtient les résultats suivants :

| | |
|---|---|
| TT de l'acide 2-méthyl-glutarique | 13 % |
| RT en acide adipique | 30 % |

### EXEMPLE 4.

On répète l'exemple 3, dans les mêmes conditions et avec les mêmes quantités des mêmes réactifs, mais sans raccorder l'autoclave à l'alimentation en monoxyde de carbone.

On obtient les résultats suivants :

| | |
|---|---|
| TT de l'acide 2-méthyl-glutarique | 60 % |
| RT en acide adipique | 11 % |

On note l'obtention d'une très forte quantité de monoacides à 5 atomes de carbone (acides valérique et méthyl-butanoïque notamment).

### EXEMPLE 5.

Dans une ampoule de verre de 50 cm³, préalablement purgé à l'argon, on introduit successivement :
- [lrCl(cod)]₂: 0,21 mmol
- iodure d'hydrogène (en solution aqueuse à 57 % en poids): 0,54 mmol
- acide 2-méthyl-glutarique : 78 mmol

L'ampoule de verre est placée dans un autoclave de 125 cm³, préalablement purgé à l'argon. L'autoclave est fermé, placé dans un four agité et raccordé à l'alimentation de CO sous pression. On met 5 bar de CO à 25°C, puis on chauffe à 230°C. On ajuste la pression en température à 100 bar à l'aide de CO (ce qui correspond à 59 bar de pression partielle de CO mesurée à 25°C) et cette pression est maintenue pendant 2 heures à 230°C.

L'autoclave est ensuite refroidi, puis dégazé et le mélange réactionnel est analysé par CPG et CLHP.

On obtient les résultats suivants :

| | |
|---|---|
| TT de l'acide 2-méthyl-glutarique | 20 % |
| RT en acide adipique | 37 % |

### EXEMPLE 6.

On répète l'exemple 5, dans les mêmes conditions et avec les mêmes quantités des mêmes réactifs, mais en opérant sous une pression de CO de 25 bar à 230°C au lieu de 100 bar.

On obtient les résultats suivants :

| | |
|---|---|
| TT de l'acide 2-méthyl-glutarique | 39 % |
| RT en acide adipique | 35 % |

### EXEMPLE 7.

On répète l'exemple 5, dans les mêmes conditions et avec les mêmes quantités des mêmes réactifs, mais en opérant sous une pression de CO de 25 bar à 230°C au lieu de 100 bar et en laissant réagir pendant 30 min en température au lieu de 2 h.

On obtient les résultats suivants :

| | |
|---|---|
| TT de l'acide 2-méthyl-glutarique | 9 % |
| RT en acide adipique | 52 % |

### EXEMPLE 8.

On répète l'exemple 5, dans les mêmes conditions et avec les mêmes quantités des mêmes réactifs, mais en opérant sous une pression de CO de 10 bar à 230°C au lieu de 100 bar.

On obtient les résultats suivants :

| | |
|---|---|
| TT de l'acide 2-méthyl-glutarique | 62 % |
| RT en acide adipique | 20 % |

### EXEMPLE 9.

On répète l'exemple 5, dans les mêmes conditions et avec les mêmes quantités des mêmes réactifs, mais en opérant à une température de 200°C au lieu de 230°C.

On obtient les résultats suivants :

| | |
|---|---|
| TT de l'acide 2-méthyl-glutarique | 3 % |
| RT en acide adipique | 64 % |

### EXEMPLE 10.

On répète l'exemple 6, dans les mêmes conditions et avec les mêmes quantités des mêmes réactifs, mais en opérant à une température de 200°C au lieu de 230°C.

On obtient les résultats suivants :

| | |
|---|---|
| TT de l'acide 2-méthyl-glutarique | 5 % |
| RT en acide adipique | 73 % |

### EXEMPLE 11.

On répète l'exemple 8, dans les mêmes conditions et avec les mêmes quantités des mêmes réactifs, mais en opérant à une température de 200°C au lieu de 230°C.

On obtient les résultats suivants :

| | |
|---|---|
| TT de l'acide 2-méthyl-glutarique | 6 % |
| RT en acide adipique | 83 % |

### EXEMPLE 12.

On répète l'exemple 6, dans les mêmes conditions et avec les mêmes quantités des mêmes réactifs, mais en chargeant en outre 7,25 mmol d'eau initialement.

On obtient les résultats suivants :

| | |
|---|---|
| TT de l'acide 2-méthyl-glutarique | 15 % |
| RT en acide adipique | 41 % |

### EXEMPLE 13.

On répète l'exemple 5, dans les mêmes conditions et avec les mêmes quantités des mêmes réactifs, mais en remplaçant HI par 0,54 mmol de HBr à 47 % en poids dans l'eau.

On obtient les résultats suivants :

| | |
|---|---|
| TT de l'acide 2-méthyl-glutarique | 7 % |
| RT en acide adipique | 53 % |

### EXEMPLE 14.

On répète l'exemple 6, dans les mêmes conditions et avec les mêmes quantités des mêmes réactifs, mais en remplaçant HI par 0,54 mmol de HBr à 47 % en poids dans l'eau.

On obtient les résultats suivants :

| | |
|---|---|
| TT de l'acide 2-méthyl-glutarique | 17 % |
| RT en acide adipique | 25 % |

### EXEMPLE 15.

On répète l'exemple 8, dans les mêmes conditions et avec les mêmes quantités des mêmes réactifs, mais en remplaçant HI par 0,54 mmol de HBr à 47 % en poids dans l'eau.

On obtient les résultats suivants :

| | |
|---|---|
| TT de l'acide 2-méthyl-glutarique | 24 % |
| RT en acide adipique | 18 % |

### EXEMPLE 16.

Dans une ampoule de verre de 50 cm³, préalablement purgé à l'argon, on introduit successivement :
- [IrCl(cod)]₂ : 0,21 mmol
- iodure d'hydrogène (en solution aqueuse à 57 % en poids) : 0,30 mmol
- acide 2-éthyl-succinique : 10 mmol
- acide acétique : 10 cm³

On opère comme dans l'exemple 5, avec une pression de CO en température de 25 bars et en maintenant l'essai à 230°C pendant 5 heures.

On obtient les résultats suivants :

| | |
|---|---|
| TT de l'acide 2-éthyl-succinique | 26 % |
| RT en acide adipique | 47 % |
| RT en acide 2-méthyl-glutarique | 19 % |

## Revendications

1. Procédé d'isomérisation par chauffage, d'au moins un acide carboxylique saturé ramifié, caractérisé en ce que l'on opère en présence d'une quantité efficace d'un catalyseur à l'iridium et d'un promoteur iodé ou bromé, le rapport molaire promoteur/lr étant compris entre 0,1/1 et 20/1.

2. Procédé selon la revendication 1, caractérisé en ce que l'acide carboxylique saturé ramifié mis en oeuvre est choisi parmi l'acide 2-méthyl-glutarique, l'acide 2-éthyl-succinique et leurs mélanges entre eux et/ou avec d'autres acides carboxyliques ou lactones formés en même temps qu'eux, comme l'acide adipique, les acides penténoïques, l'acide valérique, la gamma-valérolactone.

3. Procédé selon la revendication 2, caractérisé en ce que dans le mélange mis en oeuvre, les composés autres que l'acide 2-méthyl-glutarique et l'acide 2-éthyl-succinique représentent jusqu'à 50 % en poids du poids total du mélange.

4. Procédé selon l'une des revendications 1 à 3, caractérisé en ce que le catalyseur à l'iridium mis en oeuvre est choisi parmi :
- Ir métallique; IrO₂ , lr₂O₃ ;
- IrCl₃ ; lrCl₃, 3H₂O ;
- lrBr₃ ; lrBr₃, 3H₂O ;
- lrl₃ ;
- lr₂(CO)₄Cl₂ ; lr₂(CO)₄l₂ ;
- lr₂(CO)₈ ; lr₄(CO)₁₂ ;
- Ir(CO)[P(C₆H₅)₃]₂l ;
- lr(CO)[P(C₆H₅)₃]₂Cl ;
- lr[P(C₆H₅)₃]₃l ;
- Hlr[P(C₆H₅)₃]₃(CO) ;
- lr(acac)(CO)₂ ;
- [lrCl(cod)]₂ .

5. Procédé selon l'une des revendications 1 à 4, caractérisé en ce que la quantité de catalyseur, exprimée en moles d'iridium métal par litre de mélange réactionnel, est comprise entre 10⁻⁴ et 10⁻¹ et de préférence est comprise entre 5.10⁻⁴ et 5.10⁻² mole/litre.

6. Procédé selon l'une des revendications 1 à 5, caractérisé en ce que le promoteur iodé ou bromé est choisi parmi l'iodure d'hydrogène, le bromure d'hydrogène et les composés organo-iodés et organo-bromés capables de générer respectivement l'iodure d'hydrogène et le bromure d'hydrogène dans les conditions réactionnelles.

7. Procédé selon l'une des revendications 1 à 6, caractérisé en ce que le rapport molaire promoteur/lr est compris entre 1/1 et 10/1.

8. Procédé selon l'une des revendications 1 à 7, caractérisé en ce que la réaction est conduite en présence de monoxyde de carbone, sous une pression partielle du monoxyde de carbone, mesurée à 25°C, de 0,5 bar à 100 bars et de préférence de 1 bar à 80 bars.

9. Procédé selon l'une des revendications 1 à 8, caractérisé en ce que la réaction est conduite en phase liquide à une température de 100°C à 300°C et de préférence de 150°C à 250°C.

10. Procédé selon l'une des revendications 1 à 9, caractérisé en ce que la réaction d'isomérisation de l'acide carboxylique est conduite dans l'acide carboxylique lui-même comme milieu liquide de la réaction.

11. Procédé selon l'une des revendications 1 à 9, caractérisé en ce que la réaction d'isomérisation de l'acide carboxylique saturé ramifié est conduite dans un tiers solvant choisi parmi le benzène, le toluène, le chlorobenzène, les acides carboxyliques aliphatiques, saturés ou insaturés, ou aromatiques, comportant au plus 20 atomes de carbone, les hydrocarbures saturés aliphatiques ou cycloaliphatiques et leurs dérivés chlorés, qui sont liquides dans les conditions réactionnelles.

12. Procédé selon la revendication 11, caractérisé en ce que le solvant représente de 10 % à 99 % en volume par rapport au volume total du mélange réactionnel et de préférence de 30 % à 90 % en volume.

13. Procédé selon l'une des revendications 1 à 12, caractérisé en ce que la réaction est conduite en présence d'eau.

14. Procédé selon l'une des revendications 1 à 13, caractérisé en ce que l'eau représente de 0 à 20 % en poids du mélange réactionnel et de préférence de 0 à 10 %.

## Patentansprüche

1. Verfahren zur Isomerisierung durch Erhitzen von zumindest einer gesättigten verzweigten Carbonsäure, dadurch gekennzeichnet, daß man in Gegenwart einer wirksamen Menge eines Iridiumkatalysators und eines brom- oder jodhaltigen Promotors arbeitet, wobei das Molverhältnis Promotor/Ir zwischen 0,1:1 und 20:1 liegt.

2. Verfahren gemäß Anspruch 1, dadurch gekennzeichnet, daß die eingesetzte gesättigte verzweigte Carbonsäure unter 2-Methylglutarsäure, 2-Ethyl-bernsteinsäure und deren Gemischen untereinander und/oder mit anderen gleichzeitig mit ihnen gebildeten Carbonsäuren oder Lactonen wie Adipinsäure, die Pentensäuren, Valeriansäure und Gamma-Valerolacton ausgewählt wird.

3. Verfahren gemäß Anspruch 2, dadurch gekennzeichnet, daß in dem eingesetzten Gemisch die anderen Verbindungen als 2-Methylglutarsäure und 2-Ethyl-bernsteinsäure höchstens 50 Gew.-%, bezogen auf das Gesamtgewicht des Gemisches, ausmachen.

4. Verfahren gemäß einem der Ansprüche 1 bis 3, dadurch gekennzeichnet, daß der eingesetzte Iridiumkatalysator ausgewählt wird unter:
- Ir metallisch; IrO₂; Ir₂O₃;
- IrCl₃ ;lrCl₃, 3H₂O ;
- IrBr₃ ; IrBr₃, 3H₂O ;
- lrl₃ ;
- lr₂(CO)₄Cl₂ ; lr₂(CO)₄l₂ ;
- lr₂(CO)₈ ; lr₄(CO)₁₂ ;
- lr(CO)[P(C₆H₅)₃]₂l ;
- Ir(CO)[P(C₆H₅)₃]₂Cl ;
- lr[P(C₆H₅)₃]₃l ;
- Hlr[P(C₆H₅)₃]₃(CO) ;
- lr(acac)(CO)₂ ;
- [IrCl(cod)]₂ .

5. Verfahren gemäß einem der Ansprüche 1 bis 4, dadurch gekennzeichnet, daß die Katalysatormenge, ausgedrückt in Mol Iridiummetall je Liter Reaktionsgemisch, zwischen 10⁻⁴ und 10⁻¹, und bevorzugt zwischen 5 x 10⁻⁴ und 5 x 10⁻² Mol/l beträgt.

6. Verfahren gemäß einem der Ansprüche 1 bis 5, dadurch gekennzeichnet, daß der jod- oder bromhaltige Promotor unter Jodwasserstoff, Bromwasserstoff und den jodorganischen und bromorganischen Verbindungen, die imstande sind, Jodwasserstoff bzw. Bromwasserstoff unter den Reaktionsbedingungen zu liefern, ausgewählt wird.

7. Verfahren gemäß einem der Ansprüche 1 bis 6, dadurch gekennzeichnet, daß das Molverhältnis Promotor/Ir zwischen 1:1 und 10:1 beträgt.

8. Verfahren gemäß einem der Ansprüche 1 bis 7, dadurch gekennzeichnet, daß die Reaktion in Anwesenheit von Kohlenmonoxid unter einem Kohlenmonoxidpartialdruck, gemessen bei 25°C, von 0,5 bar bis 100 bar und bevorzugt 1 bar bis 80 bar durchgeführt wird.

9. Verfahren gemäß einem der Ansprüche 1 bis 8, dadurch gekennzeichnet, daß die Reaktion in flüssiger Phase bei einer Temperatur von 100 bis 300°C und bevorzugt 150 bis 250°C durchgeführt wird.

10. Verfahren gemäß einem der Ansprüche 1 bis 9, dadurch gekennzeichnet, daß die Isomerisierungsreaktion der Carbonsäure in der Carbonsäure selbst als flüssigem Reaktionsmedium durchgeführt wird.

11. Verfahren gemäß einem der Ansprüche 1 bis 9, dadurch gekennzeichnet, daß die Isomerisierungsreaktion der gesättigten verzweigten Carbonsäure in einem dritten Lösungsmittel, ausgewählt unter Benzol, Toluol, Chlorobenzol, den gesättigten oder ungesättigten aliphatischen oder den aromatischen Carbonsäuren mit höchstens 20 Kohlenstoffatomen, den gesättigten aliphatischen oder cycloaliphatischen Kohlenwasserstoffen und deren chlorierten Derivaten, die unter den Reaktionsbedingungen flüssig sind, durchgeführt wird.

12. Verfahren gemäß Anspruch 11, dadurch gekennzeichnet, daß das Lösungsmittel 10 bis 99 Vol.%, bezogen auf das Gesamtvolumen des Reaktionsgemisches, und bevorzugt 30 bis 90 Vol.%, ausmacht.

13. Verfahren gemäß einem der Ansprüche 1 bis 12, dadurch gekennzeichnet, daß die Reaktion in Gegenwart von Wasser durchgeführt wird.

14. Verfahren gemäß einem der Ansprüche 1 bis 13, dadurch gekennzeichnet, daß das Wasser 0 bis 20 Gew.% des Reaktionsgemisches, und bevorzugt 0 bis 10%, ausmacht.

## Claims

1. Process for the isomerization, by heating, of at least one saturated, branched carboxylic acid, characterized in that the process is carried out in the presence of an effective quantity of an iridium catalyst and of an iodine-containing or bromine-containing promoter, the promoter/Ir molar ratio being between 0.1/1 and 20/1.

2. Process according to claim 1, characterized in that the saturated, branched carboxylic acid used is chosen from 2-methylglutaric acid, 2-ethylsuccinic acid and their mixtures with one another and/or with other carboxylic acids or lactones formed at the same time as these acids, such as adipic acid, the pentenoic acids, valeric acid and gamma-valerolactone.

3. Process according to ciaim 2, characterized in that, in the mixture used, the compounds other than 2-methylglutaric acid and 2-methylsuccinic acid represent up to 50% by weight of the total weight of the mixture.

4. Process according to one of claims 1 to 3, characterized in that the iridium catalyst used is chosen from :
- Ir metal; IrO₂ ; Ir₂O₃ ;
- IrCl₃ ; IrCl₃.3H₂O ;
- IrBr₃ ; IrBr₃.3H₂O ;
- IrI₃ ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂I ;
- Ir(CO)[P(C₆H₅)₃]₂Cl ;
- Ir(P(C₆H₅)₃]₃I ;
- HIr[P(C₆H₅)₃]₃(CO) ;
- Ir(acac)(CO)₂ ;
- [IrCl(cod)]₂.

5. Process according to one of claims 1 to 4, characterized in that the quantity of catalyst, expressed in moles of iridium metal per litre of reaction mixture, is between 10⁻⁴ and 10⁻¹, and is preferably between 5x10⁻⁴ and 10⁻² mol/litre.

6. Process according to one of claims 1 to 5, characterized in that the iodine-containing or bromine-containing promoter is chosen from hydrogen iodide, hydrogen bromide and the organoiodine and organobromine compounds which are capable of generating respectively hydrogen iodide and hydrogen bromide under the reaction conditions.

7. Process according to one of claims 1 to 6, characterized in that the promoter/Ir molar ratio is between 1/1 and 10/1.

8. Process according to one of claims 1 to 7, characterized in that the reaction is carried out in the presence of carbon monoxide, at a partial pressure of carbon monoxide, measured at 25°C, of 0.5 bar to 100 bars, and preferably from 1 bar to 80 bars.

9. Process according to one of claims 1 to 8, characterized in that the reaction is carried out in the liquid phase at a temperature of 100°C to 300°C, and preferably from 150°C to 250°C.

10. Process according to one of claims 1 to 9, characterized in that the isomerization reaction of the carboxylic acid is carried out in the carboxylic acid itself as liquid reaction medium.

11. Process according to one of claims 1 to 9, characterized in that the isomerization reaction of the saturated branched carboxylic acid is carried out in a dissolving intermediary chosen from benzene, toluene, chlorobenzene, saturated or unsaturated aliphatic or aromatic carboxylic acids containing not more than 20 carbon atoms, saturated aliphatic or cycloaliphatic hydrocarbons and their chlorinated derivatives which are liquid under the reaction conditions.

12. Process according to claim 11, characterized in that the solvent represents from 10% to 99% by volume relative to the total volume of the reaction mixture, and preferably from 30% to 90% by volume.

13. Process according to one of claims 1 to 12, characterized in that the reaction is carried out in the presence of water.

14. Process according to one of claims 1 to 13, characterized in that the water represents from 0 to 20% by weight of the reaction mixture, and preferably from 0 to 10%.
